# EUROPEAN PATENT APPLICATION

(11) **EP 2 439 669 A1**
(43) Date of publication of application: **11.04.2012**
(21) Application number: 11184382.7
(22) Date of filing: 07.10.2011
(51) Int. Cl.: G06F 19/00

(54) **Computer-implemented system and method for facilitating patient advocacy through online healthcare provisioning**

(30) Priority: 08.10.2010 US 901433
(71) Applicant: Cardiac Science Corporation, Bothell, WA 98021 (US)
(72) Inventor: Bardy, Gust H., Carnation, WA Washington 98014 (US); Bishay, Jon Mikalson, Seattle, WA Washington 98119 (US)
(74) Representative: Curley, Donnacha John

(57) **Abstract**

A computer-implemented system and method (10) for facilitating patient advocacy through online health care is provided. A patient advocacy database (77) is maintained. General physicians (22) (22) and specialist physicians (27) are listed in database (77) records, as well as a diagnostic criteria (30) for health disorders for each specialist (27). A patient referral tree (20) is built with each general physician (22) associated with specialists. A medical service network (72) includes the referral tree (20) as designating health care and medical service providers. A patient (41) is enrolled in the network (72). Medical data (51) provided by the patient (41) is evaluated against the diagnostic criteria (30) of each of the specialists for medical concerns. Each specialist in the referral tree (20) corresponding to findings made under their respective diagnostic criteria (30) is identified. The patient (41) is referred for care to the specialist associated with the patient's general physician (22). Throughout, the patient (41) is provided with information to make an informed decision with respect to the specialist care received.

## Description

### Field

This application relates in general to online health care management and, in particular, to a computer-implemented system and method for facilitating patient advocacy through online health care provisioning.

### Background

Managed health care attempts to reduce the costs of benefits and services, while improving health care quality. These goals are often in opposition. Generally, managed care plans require patients to see their primary care physician first and diagnostic testing is limited to routine health problems falling within the knowledge base of the primary care physicians. In-depth testing requires referral to medical specialists who are best suited to evaluate specialized medical need.

Getting in-depth testing and access to specialist care can be difficult under managed care for reasons less related to cost constraints than to the time, focus, and knowledge of the primary care physicians, who typically see up to 50 patients per day complaining of a wide range of concerns. The pressures of patient throughput and the need to avoid unnecessary and costly referrals are two prominent disincentivizes for primary care physicians to be proactive in pursuing non-routine specialized patient care. For instance, health disorders with sporadic or intermittent symptoms, such as cardiac arrhythmias, are usually asymptomatic and are generally not present during a medical appointment. Sporadic disorders can be difficult to diagnose and for non-expert physicians to effectively manage. Consequently, these kinds of complaints may well be written off by primary care physicians as either non-existent or as originating from benign causes, as both rationales are easy to justify when the external pressures to write-off patient complaints are omnipresent. Thus, the patient is forced to see his primary care physician repeatedly, if he has the stamina and knowledge, until the concern is addressed, or, as often happens, to seek medical attention through emergency care facilities. Time, money, effort, and days off from work are needlessly expended. Worse yet, death may occur from failure to obtain the appropriate in-depth tests.

Simple procedures by primary care providers could help resolve patient access issues if only the process for tests, subsequent interpretation, and specialist referral were less onerous. For example, in the case of patients that might have cardiac rhythm abnormalities, ambulatory electrocardiographic (ECG) monitoring is used to collect cardiac data over an extended period while a patient engages in activities of daily living. Conventionally, a patient must first see his primary care provider who, at his discretion, evaluates the medical necessity for such a test. If Holter monitoring is ordered, both the doctor and the patient must make a testing laboratory appointment to have the monitor placed, return to the laboratory to have the monitor removed following monitoring, and await testing results. The primary care provider must receive the test results, which is not a given condition, review any diagnostic findings, be informed about what the test results show, also not a given condition, consult with a cardiac specialist, yet another uncertain link in the chain of evaluation, and then decides whether referral or further follow up to that specialist is needed. Each step adds an additional 30 percent of administrative overhead costs on average, plus requires hours of the physicians' and patient's time. Little of this effort is physician-reimbursed, which can be a financial drain on the primary care physician's practice. The incentive to forego action is strong, not only financially, but due to time constraints, ignorance, and cognitive overload. Not surprisingly, the majority of patient complaints regarding their heart rhythm go unaddressed.

Each task involved in assessing whether a patient has a cardiac rhythm disorder also involves separate health care entities, including the primary care provider, the Holter monitor laboratory, the cardiac specialist charged with diagnostic over read of monitoring results, and follow up by a cardiac specialist possibly different than the cardiac specialist who performed the over read. At a minimum, multiple information exchanges and patient permissions are required and travel and multiple appointments must be undertaken before diagnostic follow up is complete. These steps entail additional time, expense, and resources, and only serve to frustrate patients by creating unnecessary friction, anxiety, and waiting. Moreover, primary care providers receive minimal to no reimbursement for overseeing completion of these steps, most of which are ancillary to his primary medical care function. In short, evaluation of the common problem of cardiac rhythm disorders is rarely performed satisfactorily, or at all.

Alternatively, a patient could embark on self-care through the emergency room or by consulting with an out-of-network provider. Without the primary care provider's involvement, though, the patient faces an uphill battle in navigating through the managed care system and is at risk of non-reimbursement if care is sought outside of the managed care network or is for services not recognized as originating with the primary care physician. The patient is ultimately left frustrated and his medical need will likely remain unmet.

Conventional health care support services fail to adequately address these shortcomings. For instance, iTriage, a health care information service operated by Healthagen, LLC, Lakewood, CO, maintains a national directory of hospitals, urgent cares, retail clinics, pharmacies, and physicians. Individuals can access the directory using an application that executes on a mobile computing device or by using a Web browser. The service helps the individual to pinpoint symptoms and identify possible causes, then provides information that helps determine the closest physical facility most appropriate to treating the cause. However, the service operates outside of traditional managed health care. Health care providers must separately subscribe to the service to be listed and receive patient referrals.

U.S. Patent application, Publication No. 2007/0255153, filed November 1, 2007, to Kumar et al.; U.S. Patent application, Publication No. 2007/0225611, filed February 6, 2007, to Kumar et al.; and U.S. Patent application, Publication No. 2007/0249946, filed February 6, 2007, to Kumar et al. disclose a non-invasive cardiac monitor and methods of using continuously recorded cardiac data. A heart monitor suitable for use in primary care includes a self-contained and sealed housing. Continuously recorded cardiac monitoring is provided through a sequence of simple detect-store-offload operations that are performed by a state machine. The housing is adapted to remain affixed to a mammal from at least seven days up through 30 days. The heart monitor can include an activation or event notation button, the actuation of which increases the fidelity of the ECG information stored in the memory. The stored information can be retrieved and analyzed offline to identify ECG events, including determining the presence of an arrhythmia.

Finally, U.S. Patent application, Publication No. 2008/0284599, filed April 28, 2006, to Zdeblick et al. and U.S. Patent application, Publication No. 2008/0306359, filed December 11, 2008, to Zdeblick et al., disclose a pharma-informatics system for detecting the actual physical delivery of a pharmaceutical agent into a body. An integrated circuit is surrounded by pharmacologically active or inert materials to form a pill, which dissolve in the stomach through a combination of mechanical action and stomach fluids. As the pill dissolves, areas of the integrated circuit become exposed and power is supplied to the circuit, which begins to operate and transmit a signal that may indicate the type, A signal detection receiver can be positioned as an external device worn outside the body with one or more electrodes attached to the skin at different locations. The receiver can include the capability to provide both pharmaceutical ingestion reporting and psychological sensing in a form that can be transmitted to a remote location, such as a clinician or central monitoring agency.

Therefore, a need remains for a way to assist patients in receiving specialized diagnostic testing and follow up medical care within the narrow confines of managed health care and without risk of non-reimbursement.

### Summary

A computer-implemented system and method for directly serving the needs of patients is provided. Patients are enrolled into a server-based medical service network through remote computer workstations. Where available, the patient's electronic medical records are centrally consolidated, along with any medical data provided by the patient directly, subject to controls on data veracity and reliability. A geographically relevant referral tree is maintained by the medical service network in a database electronically stored and accessible through the server. The referral tree represents physician-to-physician referrals, medical specializations, and diagnostic criteria relationships within the patient's home locale. The patient-provided medical data is evaluated and a medical diagnosis is made based on any findings. The patient is referred to a medical specialist through the referral tree and via the information embedded on the appropriate diagnostic tool, such as a Holter monitor, and direct follow up is provided for the patient.

One embodiment provides a computer-implemented system and method for facilitating patient advocacy through online health care provisioning. A patient advocacy database is maintained. General physicians are listed in records in the database. Specialist physicians are listed in records in the database. A diagnostic criteria for one or more health disorders is listed in records in the database for the medical specialty of each specialist physician. A patient referral tree is built, which includes each general physician and an association with one or more of the specialist physicians. A medical service network is operated and includes the patient referral tree as designating providers of health care and medical services. A patient is enrolled in the medical service network, wherein the patient is under care of one of the general physicians. Medical data provided by the patient is evaluated against the diagnostic criteria of each of the specialist physicians for medical concerns. Each specialist physician in the patient referral tree corresponding to findings made under their respective diagnostic criteria is identified. The patient is referred for health care and medical services to the identified specialist physician that is associated with the general physician of the patient.

A further embodiment provides a computer-implemented system and method for facilitating cardiac rhythm patient monitoring and follow up care. A patient referral tree is formed. The patient referral tree includes listings of each of general physicians, cardiac specialist physicians that are associated with one or more of the general physicians, and diagnostic criteria for cardiac rhythm diseases for each of the cardiac specialist physicians. A patient is enrolled in a medical service network that includes the general and cardiac specialist physicians in the patient referral tree. The patient is monitored using an ambulatory electrocardiographic monitor applied by one such general physician. The ambulatory electrocardiographic monitor includes leadless integrated sensing electrodes and recording circuitry provided in a single-use compact disposable package. An electrocardiogram retrieved from the recording circuitry of the ambulatory electrocardiographic monitor is evaluated against the diagnostic criteria. Upon making a finding of a cardiac rhythm abnormality when at least one of the diagnostic criteria for cardiac rhythm diseases is met, cardiac specialist physicians in the patient referral tree corresponding to the finding are identified. The patient is directly referred to the identified cardiac specialist physician who is associated with the general physician of the patient.

Thus, patients who have proactively sought diagnostic testing are able to directly access specialized medical care via pre-populated relational databases, rather than the serial interactions that are required for the primary care provider. The diagnostic testing results are vetted to determine most appropriate follow up, thereby supplanting the at-times counterproductive screening ordinarily performed by their primary care provider.

Primary care physicians are empowered with a type of ambulatory ECG monitoring that, in conjunction with a referral center, ensures proper data interpretation and medical follow up. A primary care physician need only apply an ambulatory ECG monitor in-clinic or provide a monitor to a patient through a prescription called into a pharmacy or other dispensary point-of-sale. Subspecialty expertise in arrhythmia diagnosis need not be resident in the provider's clinic, nor must the patient be referred to a separate ambulatory ECG testing laboratory. The low cost of each monitor encourages use when patient symptoms urge access to ambulatory ECG monitoring data. The backup system of support for the general physician helps minimize the risk of misdiagnosis and the need to even establish a referral, which is often not a simple decision or a simple process to ensure. Additionally, the combination of low cost and convenience of access to expertise encourages testing when appropriate to evaluating new medications or other changes important for the conduct of high-quality medical care.

Another key feature is that patients are empowered with the ability to self-screen a potential arrhythmic condition through ambulatory ECG monitoring. Access to cardiac rhythm expertise is difficult for a variety of reasons. Patients save both the costs and inconvenience of undertaking intermediate diagnostic testing, as typically required when undergoing conventional Holter-type ambulatory ECG monitoring, as well as avoid the risk of non-reimbursement that arises when they seek help outside their managed care plan. Patients are able to stay informed of their test results and follow on care without having to passively wait for follow up to occur. Moreover, wasted time is avoided by all interested parties.

Finally, as part of the system employed by primary care providers, cardiac specialists are empowered with receiving complete patient referrals and critical ECG data that enable them to effectively diagnose and treat arrhythmic conditions without the usual repetitive phone calls and requests to access medical information between doctors offices. Medical information and patient-generated diary entries are communicated to the referral center as part of the ambulatory ECG monitoring process, which is provided to cardiac specialists as part of a complete referral.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, all without departing from their spirit and the scope. Accordingly, the drawings and detailed description are to be regarded as illustrative in nature and not as restrictive.

### Brief Description of the Drawings

FIGURE 1 is a flow diagram showing a computer-implemented method for facilitating patient advocacy through online health care provisioning in accordance with one embodiment.
FIGURE 2 is a flow diagram showing a routine for building a referral tree for use in the method of FIGURE 1.
FIGURE 3 is a flow diagram showing a routine for enrolling patients in a medical service network for use in the method of FIGURE 1.
FIGURE 4 is a flow diagram showing a routine for facilitating health care provisioning for use in the method of FIGURE 1.
FIGURE 5 is a block diagram showing a computer-implemented system for facilitating patient advocacy through online health care provisioning in accordance with one embodiment.

### Detailed Description

Medicine is a profession of specializations and sub-specializations. Within that system, primary care providers are the gatekeepers to entry into the various specialized fields of medical expertise. Primary care providers are typically internal medicine, family practice, and osteopathic physicians that practice general medicine. Under managed care, and in theory, these primary care physician refer patients to physicians with specific medical expertise when indicated by medical need, subject to referral guidelines dictated by managed health care organizations. In turn, in diagnosing and caring for their patients, each specialist physician applies their own diagnostic criteria based on accepted medical guidelines and their experience. Although most primary care physicians maintain a network of referral physicians covering most areas of medical specializations, from a patient's perspective, the ability to actually access a specialist is much more difficult than would appear to be the case in theory. The logistical obstacles to referral are actually larger than commonly understood and have a significant impact on whether follow up or appropriate diagnosis occurs in a timely and non-stressful manner.

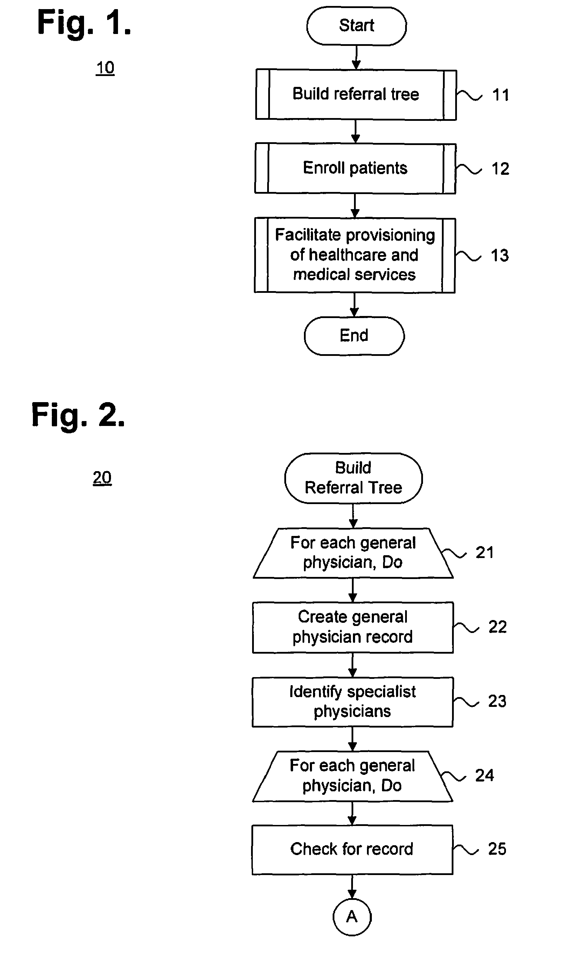

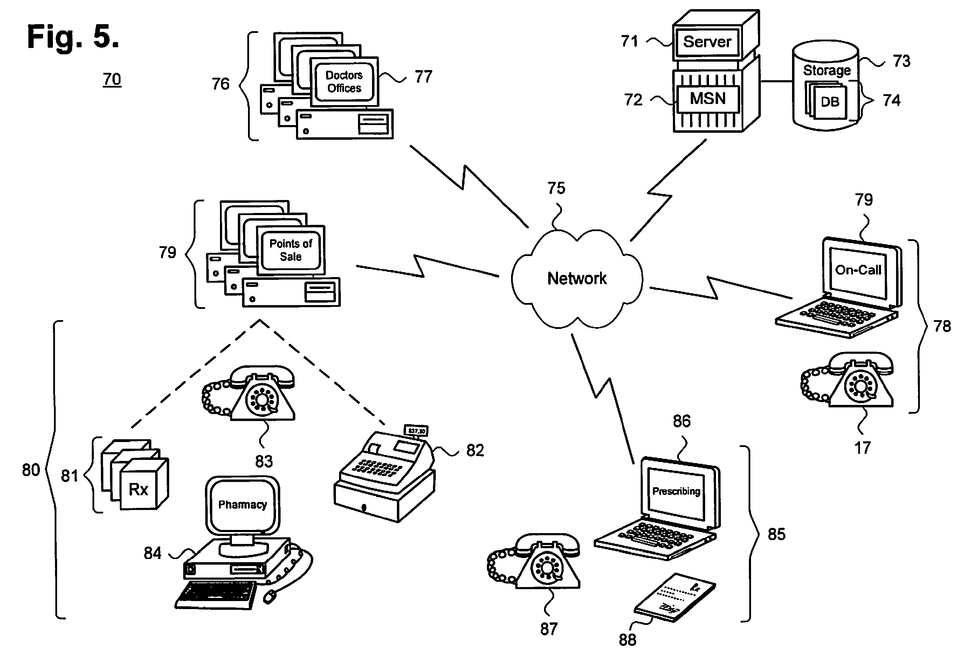

Patient confidence that their health concerns are being properly addressed can be improved by providing a surrogate to their primary care provider that could advocate for both the patient and the primary care provider and advise them when referral into medical specialization is needed. Throughout the process, the patient is provided with information to make an informed decision with respect to the specialist care sought and received. FIGURE 1 is a flow diagram showing a computer-implemented method for facilitating patient advocacy 10 through online health care provisioning in accordance with one embodiment. The method 10 operates on computing devices that include servers, personal computers, and programmable personal appliances, such as mobile telephones and digital media players, as further described below with reference to FIGURE 5. Each computing device execute modules of programmable computer code and includes those components conventionally found in general purpose programmable devices, such as a central processing unit, volatile memory, input and output ports, user display, keyboard or other input device, network interface, and non-volatile mass storage. Other components are possible.

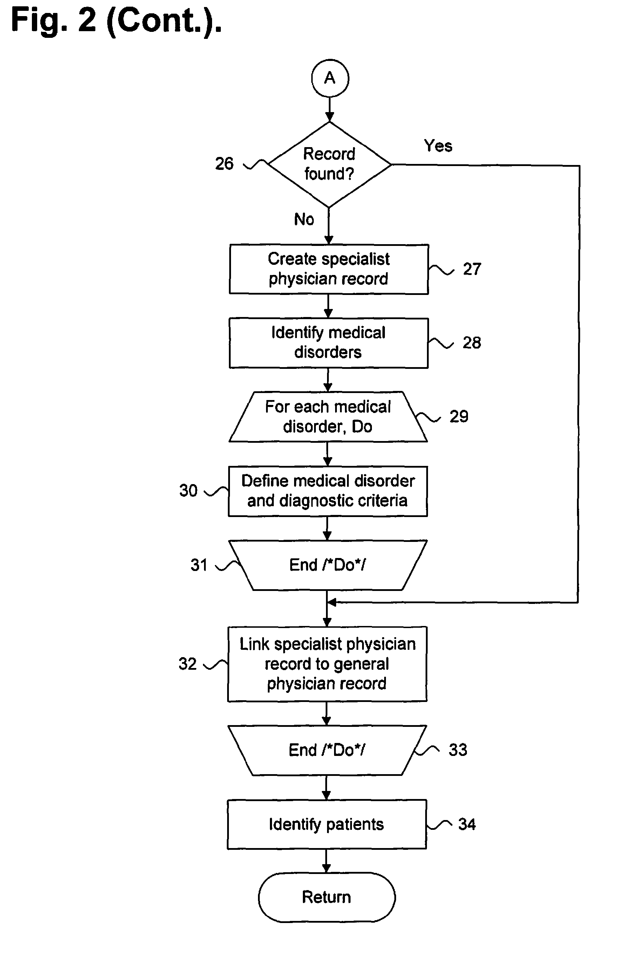

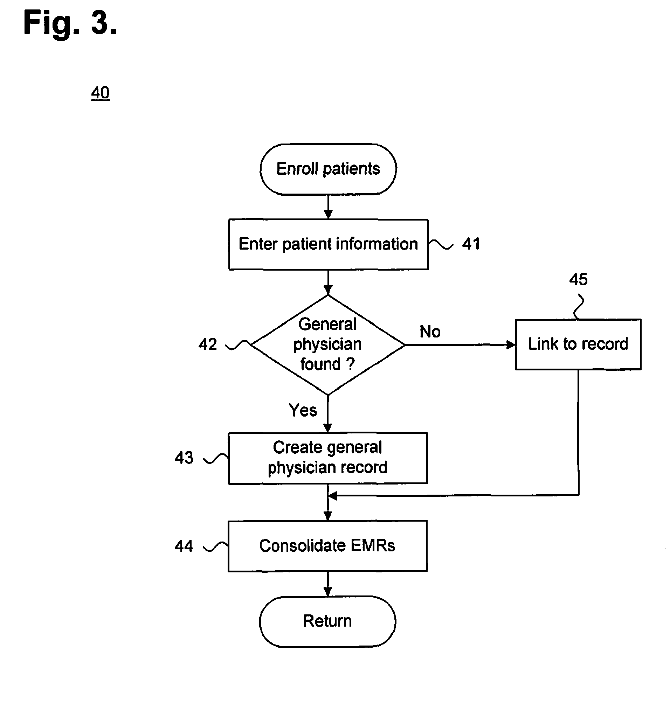

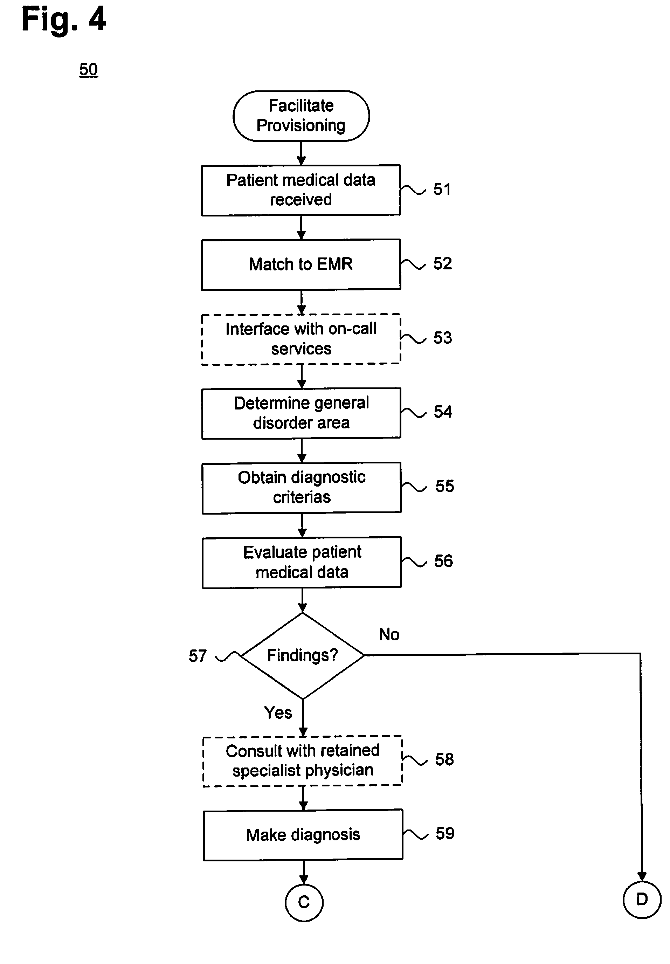

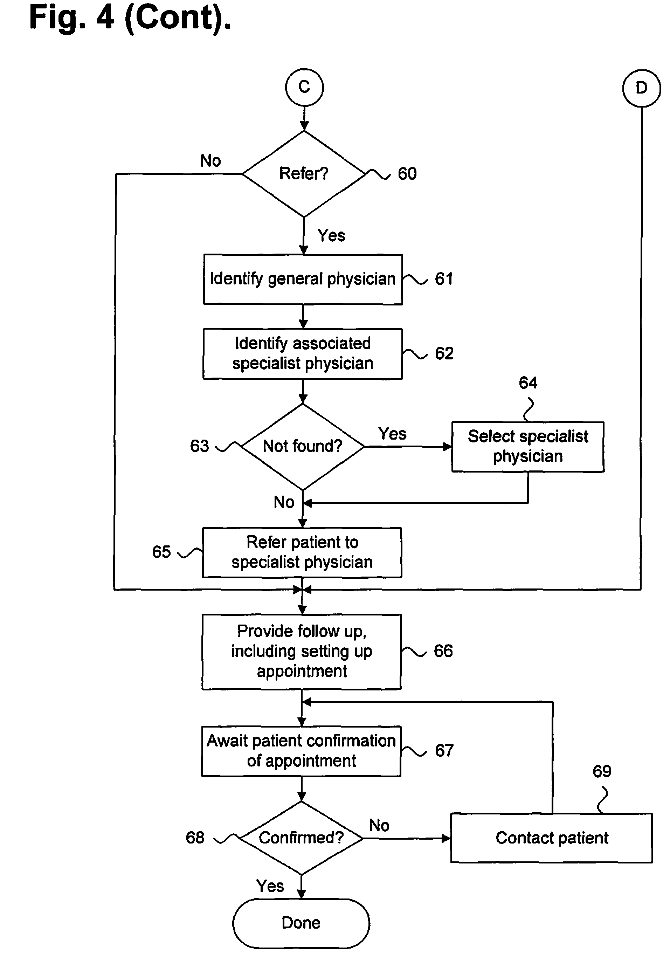

Initially, a referral tree is built (step 11) for use on a server by a medical service network that operates a monitoring, consultation, and specialist referral center ("referral center"), as further described below with reference to FIGURE 2. Patients are then enrolled into the medical service network through computer workstations remotely interfaced to the server (step 12), as further described below with reference to FIGURE 3. Finally, health care and medical service provisioning is provided to enrolled patients (step 13), as further described below with reference to FIGURE 4. The method allows 10 patients to enter into the medical service network at any point in the continuum of care provisioning without risk of denial of benefits or non-reimbursement, for instance, following self-initiated medical device monitoring, such as described in commonly-assigned U.S. Patent application, entitled "Computer-Implemented System and Method for Mediating Patient-Initiated Physiological Monitoring," Serial No. 12/901,455, filed October 8, 2010, pending, the disclosure of which is incorporated by reference.

The referral tree electronically represents physician-to-physician referral, medical specialization, and diagnostic criteria relationships. FIGURE 2 is a flow diagram showing a routine for building a referral tree 20 for use in the method of FIGURE 1. The referral tree is populated with linked records stored in a relational database, such as the Oracle Relational Database Management System, licensed by Oracle Corporation, Redwood Shores, CA.

The database is built using general physician relationships as a starting point, although other sources of relationship data could be used. Thus, each general physician in an existing managed care network is first introduced as a record (step 22) in the referral tree (steps 21-33). The specialist physicians to whom the general physician ordinarily refers patients are identified (step 23) and processed (steps 24-31). The database is checked to see if a record for the specialist physician has already been entered (step 25). If not found (step 23), a specialist physician record is created (step 27) and the type of medical disorders in which the specialist physician specializes are identified (step 28). The diagnostic criteria used by the specialist physician for each of his medical disorders is defined and entered (step 30) into the referral tree (steps 29-31). The record for the specialist physician is then linked to the general physician's record (step 32) to establish a physician-to-physician relationship. Finally, each known patient under the general physician is identified and entered into the database (step 34). In a further embodiment, the records in the database for the general and specialist physicians can be supplemented with additional information that may be helpful to potential patients or other physicians, such as education, areas of sub-specialization, insurance accepted, geographic location, ratings, cost criteria, and so forth.

The referral tree represents physician-related relationships as a foundational part of the medical service network. FIGURE 3 is a flow diagram showing a routine for enrolling patients 40 in a medical service network for use in the method 40 of FIGURE 1. Patients are normally enrolled into the network over time. For instance, a patient might be enrolled as part of a request for a medical monitoring device for self-initiated physiological monitoring. Enrollment is started by entering patient information (step 41), which includes patient identification, vital statistics, and health insurance data. Other patient information may also be included. Where a general physician for the patient is identified (step 42), the patient's record is linked to his general physician's record in the database (step 45). Otherwise, a new record for the general physician is created (step 43).

To maximize patient benefit, the medical service network centralizes whatever electronically-stored information may be available on a patient (step 44). Patient records in the database are safeguarded against unauthorized disclosure to third parties in compliance with medical information privacy laws, such as the Health Insurance Portability and Accountability Act (HIPAA) and the European Privacy Directive. Each record is assigned tiers of permissions. For instance, general and specialist physicians in the referral tree, when linked to the patient, have full permission to all the information contained in the patient's EMRs. The patient is granted partial visibility to only those sections of his EMRs that are conventionally made available to the patient. Third parties, such as sales or technical staff for the medical service network, are provided limited permissions, which exclude patient-identifiable information. Other tiers and types of permission granting schemes are possible.

The medical service network navigates or "advocates" through managed care on behalf of individual patients. FIGURE 4 is a flow diagram showing a routine for facilitating health care provisioning 50 for use in the method 40 of FIGURE 1. In effect, the medical service network can serve as a surrogate for or even supplant the role of a primary care provider in providing diagnostic follow up health care and medical services. As a result, a patient may enter into the medical service network in situations where managed care provides little, if any, structure or guidance. For instance, health care provisioning may begin upon receipt of patient medical data (step 51), including patient medical information that originates from a non-tradition medical data source. For example, a patient may undertake self-initiated ambulatory monitoring of symptoms indicating a suspected cardiac rhythm disorder, such as *supra,* which falls outside the care of his primary care provider, using, for instance, an ambulatory ECG monitor, such as described in commonly-assigned U.S. Patent application, entitled "Ambulatory Electrocardiographic Monitor and Method of Use," Serial No. 12/901,444, filed October 8, 2010, pending; and commonly-assigned U.S. Patent application, entitled "Ambulatory Electrocardiographic Monitor for Providing Ease of Use in Women and Method of Use," Serial No. 12/901,428, filed October 8, 2010, pending, the disclosures of which are incorporated by reference. Patients are thus provided with an intermediate option of self-screening those types of health conditions that may not warrant, or that the patient chooses to forgo, primary health care provider attention without the risk of non-reimbursement. Patients avoid incurring the time, expense, and hassle of a primary care provider appointment, while indirectly eliminating the overhead charges that would be incurred under managed care.

Ordinarily, self-originated medical data could be walled off from formal medical consideration, but the medical service network allows integration of such data, subject to reasonable controls, such as acceptance of data only from validated medical devices or recognized credible and reliable sources, and will match the self-originated medical data to the patient's electronically-stored medical records (EMRs) (step 52). In a further embodiment, subjective data from the patient himself concerning his complaints at the time of occurrence can also be included with the patient's EMRs, such as diary entries created contemporaneously to ambulatory ECG monitoring, such as described in commonly-assigned U.S. Patent application, entitled "Computer-Implemented System and Method for Evaluating Electrocardiographic Ambulatory Monitoring of Cardiac Rhythm Disorders," Serial No. 12/901,461, filed October 8, 2010, pending, the disclosure of which is incorporated by reference. The diary can be implemented in the form of software, technology-assisted dictation, or conventional writing that is later electronically transcribed. Patient diary entries are helpful in temporally correlating physiological symptoms identified in ambulatory ECG data to a patient's activities of daily living and contemporaneous symptomatic complaints. In a still further embodiment, the patient medical information may include the results of repeated diagnostic testing, such as multiple sets of ECG data from ambulatory monitoring undertaken by the patient. Capture of a complete set of repeated testing results may be crucial to sequencing or trending physiological anomalies occurring over an extended period of time, or to evaluate the efficacy of changes in therapy or medication.

Similarly, a patient may have a health concern and opt to utilize "on-call" patient services (step 53), where the patient can talk to a physician, nurse practitioner, or other health care professional about their health concerns. The discussion may conventionally occur over a telephone, or be conducted electronically, including by text messaging or email. Other forms of or media for patient-to-on-call-provider interchange are possible.

The general type of medical disorder to which the received patient medical data relates is determined (step 54) and the diagnostic criteria stored in the referral tree are obtained (step 55) to evaluate the data (step 57). Where indicated by findings (step 57), the evaluation may include consultation with a physician practicing in the same medical specialization for the identified medical disorder, who has been retained by the medical service network (step 58).

A medical diagnosis is reached (step 59), either as entered into the server through a computer workstation in use by the retained specialist physician or electronically by the server itself. If indicated (step 60), a referral is electronically generated by the server as follows. First, the responsible general physician, that is, the patient's primary care provider, is identified (step 61), as well as any specialist physicians associated through the referral tree of that primary care provider (step 62). A linked specialist physician practicing in the same medical specialization for the identified medical disorder receives an automated referral for the patient (step 65). The patient is simultaneously notified via phone, email, or conventional mail that the appointment has been made and where to go and when. The specialist physician receives the patient's medical information from his EMRs, as wells as the medical diagnosis and testing results. In a further embodiment, subjective patient-created data, such as diary entries made during a monitoring session, are included with the patient medical information provided to the specialist physician. Consequently, the specialist physician receives a complete medical file for the referred patient. With a complete medical file, the physician can immediately begin assessing the patient's need and be optimally prepared to subsequently see the patient during his appointment. The physician is not disrupted by missing patient data and the patient avoids having to interact with the primary care provider and urge him to arrange for a referral. Moreover, repetitive trips to offices and repetitive phone calls usually required for scheduling his appointment, as frequently necessitated by a lack of full patient medical information, are avoided. Where no specialist physician practicing in the same medical specialization for the identified medical disorder is linked to or pre-identified by the general physician (step 63), the medical service network selects a suitable specialist physician (step 64) and makes the referral (step 65). If no referral is made because there is no compelling medical reason, the patient is flagged and follow up is undertaken with the patient's general physician to ensure the patient's perceived health concern is properly addressed.

Finally, the medical service network provides total follow up with the patient (step 66). Importantly, follow up can include automatically setting up an appointment for the patient with the referred specialist physician, including setting a date and time, when the diagnosis results in a need for specialized medical attention. The primary care physician is not a part of the referral to the specialist physician. The patient is preferably notified of the appointment through automated means, including an automated telephone call or electronic message. If a clear diagnosis is made, that is, there were no findings of a medical disorder, follow up instead includes informing the patient that their diagnosis was negative and no specialized care is needed. The medical service network ensures that the patient is aware of the follow up undertaken automatically in response to the automated diagnosis by first awaiting confirmation of the appointment (step 67) and, if necessary (step 68), contacting the patient again or through other means than originally used to provide appointment notice (step 69), such as via a manual telephone call.

As a result, the patient care loop is closed with the patient receiving guaranteed follow up, instead of being left to figure out what next steps might be required within the health care system before his medical concern is adequately answered. In addition, the patient receives an acknowledgement of the follow up from several sources, including the medical service network, the referred specialist physician, and the patient's primary care physician. This feedback loop not only accelerates patient care and avoids frustrating delays, but also ensures that patient complaints are well evaluated. Consequently, notification to the patient is generated is several forms and is communicated over different channels as necessary to ensure that the patient receives notice of required follow up. Where applicable, the patient's primary care provider is provided the diagnosis and notice of the patient's cardiac specialist appointment. The medical service network also contacts the patient to ensure he makes the appointment. Other forms of follow up are possible.

The medical service network operates as a server-based service with centralized storage and remote access to patients, physicians, and support services. FIGURE 5 is a block diagram showing a computer-implemented system 70 for facilitating patient advocacy through online health care provisioning in accordance with one embodiment. The medical service network 72 ("MSN") is a centralized online service that interfaces patients with physicians and other providers of health care and medical services through a network-connected server 71. The medical service network 19 maintains a database 74 that stores the referral tree containing the relationships of the general physicians, specialized medicine physicians, and diagnostic criteria, which is electronically maintained in storage 73 coupled to the server 71.

The medical service network 72 is connected to remote computer workstations 76, 79, 86 over a network 17. The network 17 can be either a dedicated private communications circuit or publicly-available data communications network, such as the Internet, and can include wired, wireless, or combined forms of data transmission medium. Individual physician offices and clinics 77 are interconnected through computer workstations 76 that are local to their facilities. Where medical devices 81, such as ambulatory and extended medical monitoring devices, are available to patients directly through commercial points of sale 79, such as pharmacies or authorized retail locations where medical supplies are generally sold or dispensed, each point of sale 80 typically includes a cashiering station 82, such as a cash register or point-of-sale terminal, and a telephone 83 or other type of real time communications means. Each point of sale 80 additionally includes a computer workstation 84 that is interfaced to the medical service network 72 through the server 71. Similarly, "on-call" services 78 provided to patients by the medical service network 72 typically include a telephone 80 or other type of real time communications means and a computer workstation 79. Finally, where a medical prescription issued by a licensed physician may be required for patient access to a medical device 81, one or more prescribing physicians 85 are also retained by the medical service network 72 to assist points of sale in dispensing the medical devices 81 to patients directly. Each prescribing physician 85 is interfaced over the network 75 through a computer workstation 86 to the centralized server 71. Each prescribing physician 85 also has a telephone 87 or other type of real time communications means on hand and authority to issue medical prescriptions 88. Still other components connected or interfaced directly or indirectly to the medical service network 72 are possible.

While the invention has been particularly shown and described as referenced to the embodiments thereof, those skilled in the art will understand that the foregoing and other changes in form and detail may be made therein without departing from the spirit and scope.

## Claims

1. A computer-implemented method (10) for facilitating patient advocacy through online health care provisioning, comprising:
maintaining a patient advocacy database (77), comprising:
listing general physicians (22) in records in the database (77);
listing specialist physicians (27) in records in the database (77);
listing a diagnostic criteria (30) for one or more health disorders in records in the database (77) for the medical specialty of each general physician (27);
building a patient referral tree (20) comprised of each general physician (22) and an association with one or more of the general physicians (27);
operating a medical service network (72) and comprising the patient referral tree (20) as designating providers of health care and medical services;
enrolling a patient (41) in the medical service network (72), wherein the patient (41) is under care of one of the general physicians (22);
evaluating medical data (51) provided by the patient (41) against the diagnostic criteria (30) of each of the general physicians (27) for medical concerns;
identifying each general physician (27) in the patient referral tree (20) corresponding to findings made under their respective diagnostic criteria (30); and
referring the patient (41) for health care and medical services to the identified general physician (27) that is associated with the general physician (22) of the patient (41).

2. A method according to Claim 1, further comprising:
determining one or more other identified general physicians (27) when the general physician (22) of the patient (41) lacks an association in the patient referral tree (20) to the general physicians (27) for the medical specialty matched by the findings made; and
referring the patient (41) for the health care and medical services to the other identified general physicians (27).

3. A method according to Claim 2, further comprising:
applying a selection criteria in the determination of the other general physicians (27), wherein the selection criteria comprises one of round robin and first-to-respond, education, areas of sub-specialization, insurance accepted, geographic location, ratings, and cost criteria.

4. A method according to any one of Claims 1 to 3, further comprising:
providing a call center staffed by health care professionals in communication with the medical service network (72); and
referring the patient (41) to the call center for the health care and medical services.

5. A method according to any preceding Claim, further comprising:
designating the general physicians (27) for patient triage based on urgency of medical need and their respective medical specialty; and
temporally ordering the referral of the patient (41) in accordance with the designated patient triage relative to other patients (41) also enrolled in the medical service network (72).

6. A method according to any preceding Claim, further comprising:
setting an appointment for the patient (41), including setting a date and time, with the identified general physician (27);
providing the appointment to the patient (41) as an electronically-originated message;
notifying online both the identified referral physician and the general physician (22) of the appointment for the patient (41); and
tracking the patient (41) to ensure compliance with the appointment, including setting a date and time.

7. A method according to any preceding Claim, wherein the medical service network (72) further comprises one or more on-call prescribing physicians, further comprising:
offering by-prescription-only medical devices at one or more points of prescriptive medicine dispensing;
upon a request by the patient (41) for one of the medical devices at one such point of prescriptive medicine dispensing, interfacing the patient (41) in real time with one of the on-call prescribing physicians; and
upon prescribing physician approval comprising a medical prescription dispensing the requested medical device to the patient (41).

8. A method according to any preceding Claim, wherein the medical service network (72) further comprises one or more on-call prescribing physicians, further comprising:
offering over-the-counter medical devices at one or more points of prescriptive medicine dispensing;
upon a request by the patient (41) for one of the medical devices at one such point of prescriptive medicine dispensing, interfacing the patient (41) in real time with one of the on-call prescribing physicians; and
upon prescribing physician approval comprising medical advice regarding use of the requested medical device, dispensing the device to the patient (41).

9. A method according to any preceding Claim, wherein the medical data (51) comprises electronically-stored medical history for the patient (41), further comprising including the medical history in the evaluation.

10. A method according to any preceding Claim, wherein the medical service network (72) further comprises one or more consulting physicians, further comprising:
providing the medical data (51) to one of the consulting physicians; and
receiving an electronically-stored medical diagnosis from the consulting physician and including the medical diagnosis with the findings.

11. A computer-implemented method for facilitating cardiac rhythm patient monitoring and follow up care, comprising:
forming a patient referral tree (20) comprising listings of each of general physicians (22), cardiac general physicians (27) that are associated with one or more of the general physicians (22), and diagnostic criteria (30) for cardiac rhythm diseases for each of the cardiac general physicians (27);
enrolling a patient (41) in a medical service network (72) comprised of the general and cardiac general physicians (27) in the patient referral tree (20);
monitoring the patient (41) using an ambulatory electrocardiographic monitor applied by one such general physician (22), the ambulatory electrocardiographic monitor comprising leadless integrated sensing electrodes and recording circuitry provided in a single-use compact disposable package;
evaluating an electrocardiogram retrieved from the recording circuitry of the ambulatory electrocardiographic monitor against the diagnostic criteria (30);
upon making a finding of a cardiac rhythm abnormality when at least one of the diagnostic criteria (30) for cardiac rhythm diseases is met, identifying cardiac general physicians (27) in the patient referral tree (20) corresponding to the finding; and
directly referring the patient (41) to the identified cardiac general physician (27) who is associated with the general physician (22) of the patient (41).

12. A method according to Claim 11, further comprising:
including non-cardiac general physicians (27) that are associated with one or more of the general physicians (22) and diagnostic criteria (30) for non-cardiac rhythm diseases for each of the non-cardiac general physicians (27);
upon making a finding of a non-cardiac rhythm abnormality when at least one of the diagnostic criteria (30) for non-cardiac rhythm diseases is met, identifying the non-cardiac general physicians (27) in the patient referral tree (20) corresponding to the finding; and
directly referring the patient (41) to the identified non-cardiac general physician (27) who is associated with the general physician (22) of the patient (41).

13. A method according to Claim 11, further comprising:
directly referring the patient (41) to a physician that is not included in the patient referral tree (20) upon making a finding of a non-cardiac rhythm abnormality.

14. A computer-implemented system for facilitating patient advocacy through online health care provisioning, comprising:
an electronically-stored patient advocacy database (77), comprising:
general physicians listings (22) in records in the database (77);
specialist physicians listings (27) in records in the database (77);
a diagnostic criteria listing (30) for one or more health disorders in records in the database (77) for the medical specialty of each general physician (27);
an electronically-stored patient referral tree (20) comprised of each general physician (22) and an association with one or more of the general physicians (27); and
a server coupled to the patient advocacy database (77) and implementing a medical service network (72) that comprises the patient referral tree (20) as designating providers of health care and medical services, comprising:
an enrollment module enrolling a patient (41) in the medical service network (72), wherein the patient (41) is under care of one of the general physicians (22);
an evaluation module evaluating medical data (51) provided by the patient (41) against the diagnostic criteria (30) of each of the general physicians (27) for medical concerns;
an identification module identifying each general physician (27) in the patient referral tree (20) corresponding to findings made under their respective diagnostic criteria (30); and
a referral module referring the patient (41) for health care and medical services to the identified general physician (27) that is associated with the general physician (22) of the patient (41).

15. A system according to Claim 14, wherein one or more other identified general physicians (27) are determined when the general physician (22) of the patient (41) lacks an association in the patient referral tree (20) to the general physicians (27) for the medical specialty matched by the findings made; and the patient (41) is referred for the health care and medical services to the other identified general physicians (27).
